# EUROPÄISCHE PATENTSCHRIFT

(11) **EP 0 632 836 B1**
(45) Veröffentlichungstag und Bekanntmachung des Hinweises auf die Patenterteilung: **21.05.1997**
(21) Anmeldenummer: 93907759.0
(22) Anmeldetag: 24.03.1993
(51) Int. Cl.: C12P 7/38

(54) **VERFAHREN ZUR HERSTELLUNG VON 17ALPHA-HYDROXY-3-METHOXY-8,14-SECO-1,3,5(10), 9(11)ESTRATETRAEN-14-ON**
PROCESS FOR PRODUCING 17ALPHA-HYDROXY-3-METHOXY-8,14-SECO-1,3,5(10),9(11) ESTRATETRAENE-14-ONE
PROCEDE DE FABRICATION DE 17ALPHA-HYDROXY-3-METHOXY-8,14-SECO-1,3,5(10),9(11) ESTRATETRAENE-14-ONE

(30) Priorität: 28.03.1992 DE 4210706
(43) Veröffentlichungstag der Anmeldung: 11.01.1995
(73) Patentinhaber: SCHERING AKTIENGESELLSCHAFT, 13353 Berlin (DE)
(72) Erfinder: WEBER, Alfred, D-1000 Berlin 37 (DE); KENNECKE, Mario, D-1000 Berlin 33 (DE); VIDIC, Hans-Jörg, D-1000 Berlin 37 (DE)
(86) Internationale Anmeldenummer: DE9300288
(87) Internationale Veröffentlichungsnummer: WO9320222

(56) Entgegenhaltungen:
- DD-A- 300 584
- FR-A- 1 565 986
- US-A- 3 201 324

## Beschreibung

Die Erfindung betrifft ein Verfahren zur Herstellung von 17α-Hydroxy-3-methoxy-8,14-seco-1,3,5(10),9(11)estratetraen-14-on durch Fermentation von 3-Methoxy-8,14-seco-1,3,5(10),9(11)-estratetraen-14,17-dion mit einer lebenden Kultur von Kloeckera magna.

Ein derartiges Verfahren ist bereits aus der Deutschen Patentanmeldung DE-A 17 68 513 vorbekannt. Den Ausführungsbeispielen dieser Patentanmeldung kann man entnehmen, daß dieses vorbekannte Verfahren in der Weise durchgeführt wird, daß man das Substrat in Ethanol löst und die erhaltene Lösung umsetzt.

Es wurde nun gefunden, daß es überraschenderweise möglich ist, das Substrat innerhalb signifikant kürzerer Fermentationszeit unter Erzielung siginifikant höherer Ausbeuten umzuwandeln, wenn man dem Fermentationsansatz das Substrat in Form einer lösungsmittelfreien wässrigen Suspension mit einer mittleren Korngröße von 0,2 bis 5 µm zusetzt.

Das erfindungsgemäße Verfahren wird unter den Fermentationsbedingungen durchgeführt, welche man auch bei den bekannten mikrobiologischen Umwandlungen von Substraten mit Kloeckera magna Kulturen anwendet.

Unter den für diesen Mikroorganismus üblicherweise verwendeten Kulturbedingungen werden in einem geeigneten Nährmedium unter Belüften Submerskulturen angezüchtet. Dann setzt man der Kultur eine wässrige Suspension des 3-Methoxy-8,14-seco-1,3,5(10),9(11)-estratetraen-14,17-dions zu und fementiert bis eine maximale Substratumwandlung erreicht ist.

Die wässrige Suspension des 3-Methoxy-8,14-seco-1,3,5(10),9(11)-estratetraen-14,17-dions läßt sich in einfacher Weise herstellen, indem man beispielsweise dieser Substanz mit der zweifachen bei fünffachen Menge Wasser unter Zusatz eines nichtionischen Tensids in einer Kugelmühle mahlt bis sie eine mittlere Korngröße von 0,2 bis 5 µm besitzt. Geeignete Tenside sind beispielsweise Ethylenoxidaddukte oder Fettsäureester von Polyglykolen. Als geeignete Tenside seien beispielsweise die handelsüblichen Netzmittel wie Tegin ®, Tween®, oder Span® genannt.

Nach erfolgter Fermentation wird die Kultur in üblicher Weise aufbereitet, beispielsweise indem man sie mit einem in Wasser schwer löslichen Alkohol, Keton oder Ester extrahiert, den Extrakt einengt und den erhaltenen Rückstand durch Chromatographie und/oder Kristallisation aufreinigt.

Die Weiterverarbeitung des erhaltenen 17α-Hydroxy-3-methoxy-8,14-seco-1,3,5(10),9(11)-estratetraen-14-on zu pharmakologisch wirksamen Steroiden ist bekannt oder erfolgt nach an sich bekannten Verfahren (Deutsche Patentanmeldung DE-A 17 68 513).

Das nachfolgende Ausführungsbeispiel dient zur näheren Erläuterung des erfindungsgemäßen Verfahrens.

### Beispiel:

a) Ein 2l Erlenmeyer mit 1 l sterilem Nährmedium enthaltend

| | |
|---|---|
| 0,1 % | Hefeextrakt |
| 0,1 % | Fleischextrakt |
| 0,2 % | Tryptose |
| 1,0 % | Glucose |
| -eingestellt auf pH 7,2- | |

wird mit 5 ml einer Suspension einer Kloeckera magna ATCC 20109 - Kultur beimpft und 72 Stunden bei 30 °C mit 180 Umdrehungen pro Minute geschüttelt.
b) Ein 5,5 m³ -Fermenter mit 5 m³ steriler Nährlösung enthaltend

| | |
|---|---|
| 5 % | Glucose |
| 2 % | Cornsteep liqour |
| -eingestellt auf pH 5,4- | |

wird mit 1 l der Kloeckera magna-Anzuchtkultur beimpft und 24 Stunden bei 29 °C unter Belüftung von 45 m³ pro Stunde und Rühren mit 60 Umdrehungen pro Minute inkubiert.
c) 100 kg 3-Methoxy-8,14-seco-1,3,5(10),9(11)-estratetraen-14,17-dion werden in einer Dynomühle (Firma Netzsch, Typ KD 45) mit Glaskugeln, 400 1 Wasser und 10 l Tween 80 auf eine Teilchengröße von ca. 1 µm vermalen.
d) Ein 64 m³ -Fermenter mit 50 m³ steriler Nährlösung wie unter b) angegeben wird mit 5 m³ der Kloeckera magna-Vorkultur beimpft und unter Belüftung von 500 m³ pro Stunde und Rühren mit 40 Umdrehungen pro Minute bei 29 °C 6 Stunden inkubiert.
Dann setzt man der Kultur die gemäß c) hergestellte 3-Methoxy-8,14-seco-1,3,5(10),9(11)-estratetraen-14,17-dion-Suspension zu und fermentiert weitere 24 Stunden lang unter Rühren mit 40 Umdrehungen pro Minute und einer Belüftung von 500 m³ pro Stunde.
e) Nach erfolgter Fermentation wird die Kulturbrühe dreimal mit Methylisobutylketon extrahiert, der Extrakt bei max. 50 °C unter Vakuum eingeengt. Anschließend erfolgt eine Reinigung durch Kristallisation aus Ethanol.
Man erhält 80 kg 17α-Hydroxy-3-methoxy-8,14-seco-1,3,5(10),9(11)estratetraen-14-on vom Schmelzpunkt 102-104°C.

## Patentansprüche

1. Verfahren zur Herstellung von 17α-Hydroxy-3-methoxy-8,14-seco-1,3,5(10),9(11)-estratetraen-14-on durch Fermentation von 3-Methoxy-8,14-seco-1,3,5(10),9(11)-estratetraen-14,17-dion mit einer lebenden Kultur von Kloeckera magna, dadurch gekennzeichnet, daß man das Substrat in Form einer lösungsmittelfreien wässrigen Suspension mit einer mittleren Korngröße von 0,2 bis 5 µm umsetzt.

2. Verfahren zur Herstellung von 17α-Hydroxy-3-methoxy-8,14-seco-1,3,5(10),9(11)-estratetraen-14-on gemäß Patentanspruch 1, dadurch gekennzeichnet, daß man die Fermentation mit einer lebenden Kultur von Kloeckera magna ATCC 20109 durchführt.

## Claims

1. Process for the production of 17α-hydroxy-3-methoxy-8,14-seco-1,3,5(10),9(11)-oestratetraen-14-one by fermentation of 3-methoxy-8,14-seco-1,3,5(10),9(11)-oestratetraene-14,17-dione with a live culture of Kloeckera magna, characterised in that the substrate is reacted in the form of a solventless aqueous suspension having a mean particle size of from 0.2 to 5 µm.

2. Process for the production of 17α-hydroxy-3-methoxy-8,14-seco-1,3,5(10),9(11)-oestratetraen-14-one according to patent claim 1, characterised in that the fermentation is carried out with a live culture of Kloeckera magna ATCC 20109.

## Revendications

1. Procédé pour la préparation de 17α-hydroxy-3-méthoxy-8,14-séco-1,3,5(10),9(11)-estra-tétraèn-14-one par fermentation de 3-méthoxy-8,14-séco-1,3,5(10),9(11)-estra-tétraèn-14,17-dione avec une culture vivante de Kloeckera magna, caractérisé en ce qu'on fait réagir le substrat sous forme d'une suspension aqueuse sans solvant ayant une granulométrie de 0,2 à 5 µm.

2. Procédé pour la préparation de 17α-hydroxy-3-méthoxy-8,14-séco-1,3,5(10),9(11)-estra-tétraèn-14-one selon la revendication 1, caractérisé en ce que l'on met en oeuvre la fermentation avec une culture vivante de Kloeckera magna ATCC 20109.
